(19) Europäisches Patentamt
European Patent Office
Office européen des brevets

(11) Numéro de publication : 0 233 804 B1

(12) # FASCICULE DE BREVET EUROPEEN

(45) Date de publication du fascicule du brevet : **25.09.91 Bulletin 91/39**

(21) Numéro de dépôt : **87400094.6**

(22) Date de dépôt : **16.01.87**

(51) Int. Cl.$^5$ : **C07D 253/06,** C07D 405/12, C07D 405/10, C07D 409/04, C07D 405/04, C07D 401/06, A61K 31/53

(54) **Monoaryl-5 as triazinones-3 substituées en position 2, leur procédé de préparation et leur application en tant que medicaments.**

Le dossier contient des informations techniques présentées postérieurement au dépôt de la demande et ne figurant pas dans le présent fascicule.

(30) Priorité : **16.01.86 FR 8600552**

(43) Date de publication de la demande : **26.08.87 Bulletin 87/35**

(45) Mention de la délivrance du brevet : **25.09.91 Bulletin 91/39**

(84) Etats contractants désignés : **AT BE CH DE ES FR GB GR IT LI LU NL SE**

(56) Documents cités :
**EP-A- 0 052 442**
**EP-A- 0 085 227**
**EP-A- 0 099 438**
**US-A- 3 428 635**
**BULLETIN DE LA SOCIETE CHIMIQUE DE FRANCE, no. 9-10, 1973, pages 2818-2822, No. 522, Paris, FR; J.DAUNIS et al.: "Etude en série as-triazine. XII. - Action de réactifs de Grignard sur les as-triazinones-3"**
**JOURNAL OF HETEROCYCLIC CHEMISTRY, vol. 15, décembre 1978, pages 1393-1398, HeteroCorporation, Provo, US; G.WERBER et al.: "The reactivity of the A-CH=N-NR-CX-B system. 1,3,4-Oxadiazoles and 2,3,4,5-tetrahydro-1,2,4-triazin-3-ones through the 1-5 and 1-6 cyclization reactions of alpha-thienylglyoxal-monosemicarbazones"**
**THE JOURNAL OF ORGANIC CHEMISTRY; vol. 45, no. 23, 7 novembre 1980, pages 4594-4597, American Chemical Society, Easton, US; T.SASAKI et al.: "1,2,4-Triazine chemistry. 11. Dihydro-1,2,4-triazines: Structural studies on sodium borohydride reduction products from some 3-methylthio- and 3-methoxy-1,2,4-triazines"**
**PHARMAZEUTISCHE CHEMIE, E. Schröder et al., Georg Thieme Verlag (Stuttgart), 1982**

(56) Documents cités :
**Römmps Chemie-Lexicon**
**Ullmanns Encyklopädie der technischen Chemie**

(73) Titulaire : **PIERRE FABRE MEDICAMENT**
**45, Place Abel Gance**
**F-92100 Boulogne (FR)**

(72) Inventeur : **Pitet, Guy**
**3, rue de l'Aubisque**
**F-31000 Toulouse (FR)**
Inventeur : **Cousse, Henry**
**La Foun de los Nobios, Chemin de Lastinos**
**F-81100 Castres (FR)**
Inventeur : **Stenger,, Antoine**
**11, rue des Cigales**
**F-81100 Castres (FR)**
Inventeur : **Briley, Michel**
**73, rue d'Aillot**
**F-81100 Castres (FR)**
Inventeur : **Chopin, Philippe**
**98, rue d'Aillot**
**F-81100 Castres (FR)**

(74) Mandataire : **Ahner, Francis et al**
**CABINET REGIMBEAU, 26, avenue Kléber**
**F-75116 Paris (FR)**

**EP 0 233 804 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention, réalisée au Centre de Recherche P.F. MEDICAMENT, a pour objet des nouvelles as triazines douées de propriétés pharmacologiques et utiles dans le traitement des maladies du système nerveux central, telles que l'anxiété et/ou les états dépressifs.

Les as triazines connues et utiles en thérapeutique sont exclusivement des diaryl 5-6 et plus particulièrement les composés de formule générale :

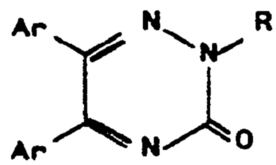

Ces composés, puissants antalgiques périphériques, sont décrits dans les brevets suivants appartenant à la demanderesse : FR-A- 2 383 176 ; FR-A- 2 500 830 FR-A- 2 544 313 et EP-A-0 099 438.

La présente invention concerne des monoaryl-5-as triazinones-3 substituées en position 2, répondant à la formule générale I :

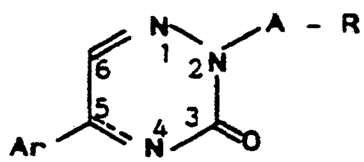

(I)

dans laquelle :

la liaison représentée par des tirets indique la présence d'une double liaison facultative ; lorsque cette double liaison n'existe pas, les atomes d'azote et de carbone occupant respectivement les positions 4 et 5 du cycle triazinique sont hydrogénés ;

A représente une liaison directe N-C, un alkylène droit ou ramifié en $C_1$ à $C_5$, éventuellement substitué une ou plusieurs fois par -COOR' ou par Ar ;

R représente

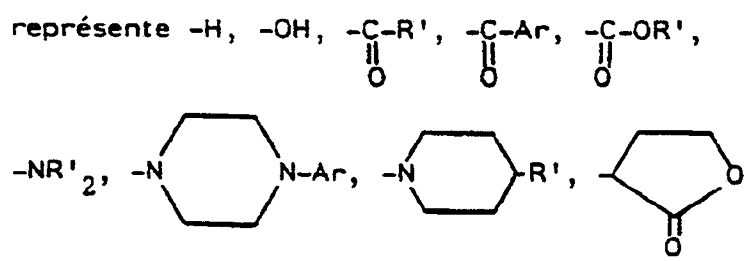

R' représente -H, alkyl droit ou ramifié en $C_1$ à $C_7$, $-NH_2$ ;

Ar représente un noyau aromatique à 5 ou 6 chaînons, contenant éventuellement un hétéroatome tel que O, N, S, et obligatoirement substitué une ou plusieurs fois par un radical choisi parmi -OH, alkyl en $C_1$ à $C_4$, alcoxy en $C_1$ à $C_4$, halogène, $-CF_3$, acétonyloxy,

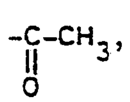

et γ butyrolactone ;

ainsi que leurs sels pharmaceutiquement acceptables.

Les composés préférés selon la présente invention sont ceux de formule générale I dans laquelle :

Ar représente

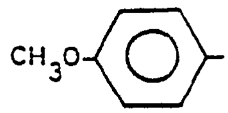

La présente invention concerne également un procédé de préparation des composés de formule générale I, caractérisé en ce qu'il répond au schéma général suivant :

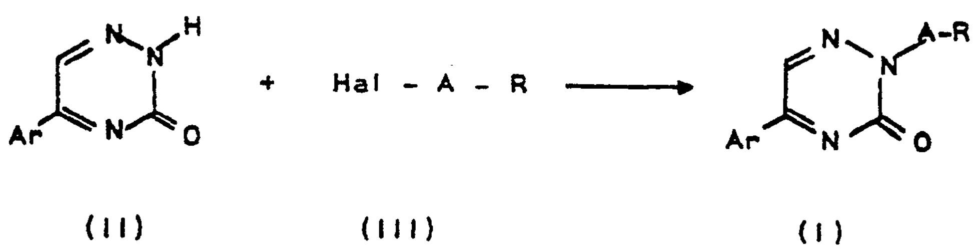

où A, R et Ar sont tels que définis précédemment

Hal est un halogène, de préférence du chlore ou du brome.

Cette réaction se déroule en présence d'un agent sodant du type hydrure ou amidure de métal alcalin. On recourt, de préférence à de l'hydrure de sodium ou de l'amidure de sodium.

On effectue cette réaction au sein d'un solvant approprié, de préférence un solvant organique, tel que le DMF.

Les intermédiaires de synthèse de formule générale (II) peuvent être obtenus par différents procédés connus dans l'état antérieur de la technique. On peut, en particulier, recourir aux techniques relatées dans les publications suivantes : R. TRUST et coll., Heterocyclic Chemistry 1979, nov., p.1393 et W.HEILMAN, J.Med-.Chem.1979,22(6) p.671.

A titre d'exemple pour la préparation de composés de formule générale II dans laquelle Ar représente

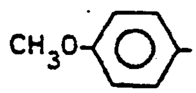

on peut procéder selon l'une des deux variantes suivantes.

Selon une première variante, le procédé de synthèse répond au schéma suivant :

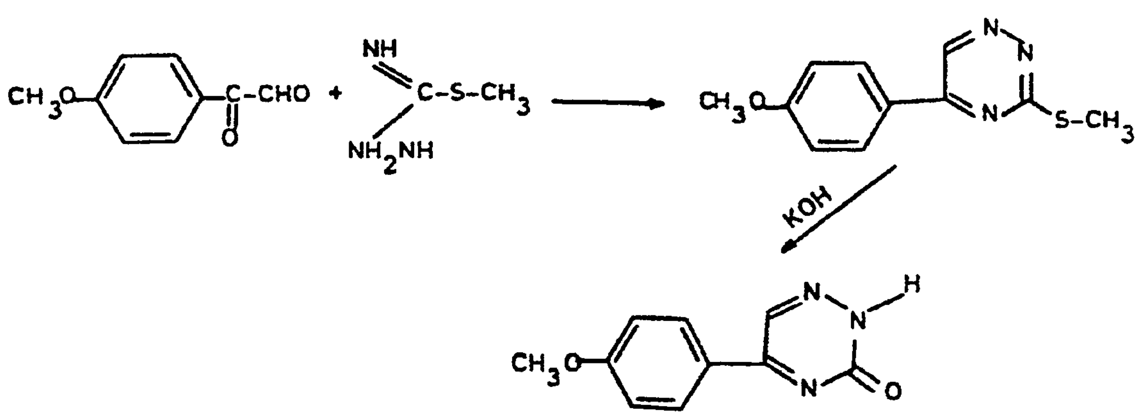


Selon cette variante, les conditions opératoires sont les suivantes :

– pour la synthèse du p-méthoxyphénylglyosal de formule développée :

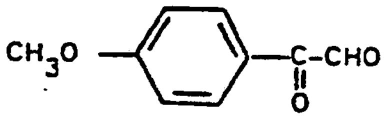

on place dans un ballon de 250 ml muni d'une agitation magnétique et d'un réfrigérant à reflux :

. 11,1 g de dioxyde de sélénium (soit $10^{-1}$ M)

. 75 ml de dioxanne

. 3 ml d'eau on chauffe à 80°C jusqu'à dissolution totale.

On ajoute directement une solution de :

. 15 g de p-méthoxyacétophénone (soit $10^{-1}$ M)

. 20 ml de dioxanne on chauffe 15 heures à reflux (la solution rougit immédiatement après l'addition).

On filtre à chaud sur buchner et lit de célite.

– pour la synthèse de la 3-méthylthio 5-p-méthoxyphényl as triazine de formule développée :

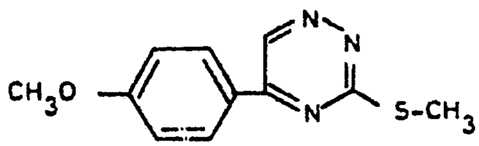

au filtrat précédemment obtenu on ajoute :

. 100 g de glace pilée

. 12,6 g de bicarbonate de sodium (soit $1,5.10^{-1}$M)

. 31 g de iodhydrate de S-méthylthiosemicarbazide (réalisée selon D.F. 375).

On abandonne sous agitation pendant 1 heure. Un produit blanc-jaune précipite, puis on laisse une nuit au réfrigérateur.

On reprend au chlorure de méthylène, lave à l'eau en ampoule, sèche sur sulfate de sodium, filtre et concentre à sec.

On obtient 16 g de produit cristallisé qui recristallise dans 10 volumes d'éthanol à 90%.

Les caractéristiques du produit obtenu sont :

P.F. : 125°C

I.R. et R.M.N. : Spectres en accord avec la structure proposée rendement : 70 à 80 %

- Enfin, pour la synthèse de oxo-3 p-méthoxyphényl-5 as triazine de formule développée :

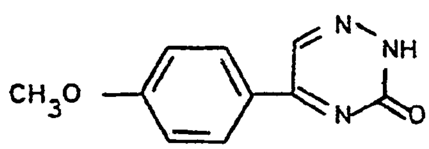

on procède de la façon suivante pour une unité opératoire de $1,7.10^{-2}$M :

Dans un ballon de 250 ml muni d'une agitation magnétique et d'un réfrigérant à reflux, surmonté d'une garde à $CaCl_2$, on place :

. 4 g de 3-méthylthio 5-p méthoxypényl as triazine

. 125 ml d'alcool éthylique séché sur tamis

. 3 g de potasse en pastilles broyées.

On chauffe 2 heures au reflux. Un produit jaune précipite, on laisse revenir à température ambiante et on refroidit à 0°C. On essore sur fritté et on lave à l'alcool éthylique.

Le précipité est dissous entièrement dans 100 ml d'eau et amené à pH 1 avec HCl concentré. Un produit blanc précipite.

On l'essore sur fritté, on le lave à neutralité à l'eau et on le sèche à l'étuve vide en présence de $P_2O_5$.

Les caractéristiques du produit obtenu sont :

P.F. : 260°C (banc Köfler)

I.R. Spectre en accord avec la structure proposée

poids : 3,12 g

rendement : 89 % (des unités opératoires de 2 à $6.10^{-2}$ M ont conduit au même rendement).

Selon une seconde variante, le procédé de synthèse des composés intermédiaires de formule générale II, dans laquelle :

Ar représente

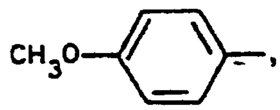

obéit au schéma réactionnel suivant :

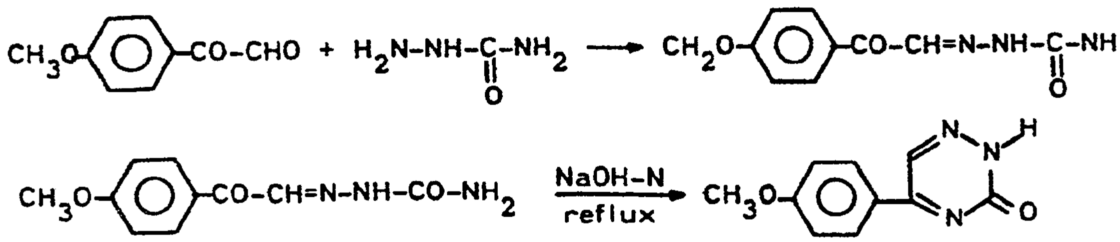


On procède de la façon suivante pour une unité opératoire de $10^{-1}$ M :

Dans un ballon de 250 ml équipé d'une agitation magnétique et d'un réfrigérant à reflux, on introduit :

. 11,1 g de dioxyde de sélénium (soit $10^{-1}$M)

. 75 ml de dioxanne

. 3 ml de $H_2O$

On chauffe à dissolution sur bain d'huile. On ajoute une solution de 15 g de p-méthoxyacétophénone (soit $10^{-1}$M) dans 20 ml de dioxanne. On chauffe 5 heures à reflux. On filtre à chaud sur buchner avec lit de célite.

On ajoute au filtrat sous agitation :

. 100 g de glace pilée

. 9 g de $NaHCO_3$

. 11,1 g de chlorhydrate de semicarbazide (soit $10^{-1}$M) (EGA)

On abandonne à vive agitation pendant 1 heure, puis 4 heures au réfrigérateur. On essore sur fritté le produit beige obtenu, on le lave abondamment à $H_2O$ et le sèche en étuve à vide en présence de $P_2O_5$.

On obtient ainsi à titre d'exemple 16,5 g de semi-carbazone correspondante qui est cyclisée par chauffage à reflux dans 200 ml de NaOH (N).

On filtre sur papier la solution chaude et on l'amène à pH acide avec 25 ml d'HCl concentré.

L'oxo-3 p-méthoxyphényl as triazine précipite. On l'essore sur fritté, on lave abondamment à l'eau et on sèche à étuve à vide en présence de $P_2O_5$.

On obtient 13,5 g d'oxo-3 p-méthoxyphényl-5 as triazine, soit un rendement de 66%.

A la suite d'expérimentations toxicologiques et pharmacodynamiques, il s'avère que les composés de formule générale I possèdent une activité thérapeutique importante, notamment dans le traitement des troubles du système nerveux central, tels que l'anxiété et/ou les états dépressifs.

C'est pourquoi la présente invention concerne des compositions thérapeutiques utiles notamment au traitement de tels troubles, et comportant au moins un composé de formule générale I à titre de principe actif, et un excipient pharmaceutiquement acceptable à titre de support ou de vecteur du principe actif.

La forme d'administration et la posologie de telles compositions pharmaceutiques dépendent du composé de formule générale I choisi, de la maladie à traiter et de l'appréciation du praticien.

Enfin, la présente invention concerne l'utilisation d'au moins l'un des composés de formule générale I ou de leurs sels, pour la préparation de composition pharmaceutiques utiles pour le traitement des maladies du système nerveux central.

Les exemples suivants permettent d'illustrer l'invention, sans aucunement en limiter la portée ; ils concernent en premier lieu les composés de formule générale I particulièrement préférés et en second lieu les résultats des expérimentations pharmacologiques entreprises sur les composés de formule générale I.

EXEMPLE 1

acétonyl-2 oxo-3 p-méthoxy phényl-5 as triazine

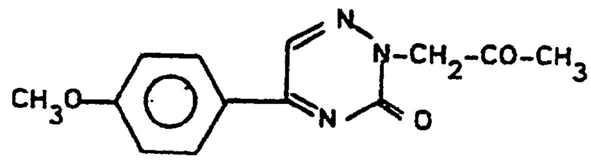

Unité opératoire : $10^{-2}$M

Dans un ballon tricol de 100 ml muni d'une agitation magnétique, d'une ampoule à introduction et d'une garde à potasse reliée à un dispositif permettant de mesurer le dégagement d'hydrogène, on place :

. 10 ml de DMF anhydre, préalablement redistillé et préservé de l'humidité sur tamis moléculaires

. 0,50 g de HNa (à 50% en dispersion dans l'huile)

On ajoute par l'ampoule, lentement, une suspension de :

. 2,03 g de triazine 3-oxo 5-p-méthoxyphényl (soit $10^{-2}$M) (synthèse n° 377)

. 50 ml de DMF

On abandonne sous agitation à température ambiante pendant environ 1 heure, jusqu'à obtention du dégagement théorique (224 ml) d'hydrogène.

On ajoute directement :

. 1,38 g de chloroacétone (soit $1,5.10^{-2}$M)

On observe une fluidification du mélange réactionnel. On l'abandonne sous agitation 3 heures à température ambiante. On concentre à sec le DMF à l'évaporateur rotatif.

On reprend le résidu par 50 ml d'eau et on extrait par 2 x 25 ml de $CH_2Cl_2$. On sèche les phases organiques sur $Na_2SO_4$ et on concentre à sec. On obtient 3 g de produit brut que l'on recristallise dans 50 ml d'EtOH. Après filtration, essorage et séchage. On obtient 2,2 g d'acétonyl-2 oxo-3 p-méthoxyphényl-5 as triazine.

Caractéristiques :

P.F.: 162°C ± 2°

I.R.: $\nu_{C=O triazine}$= 1160 (large)

$\nu_{C=O cétone}$= 1730

R.M.N. (conforme à la structure)

Rendement : 85 %

C.C.M.= Toluène-acétate d'éthyle 7:3

Rf.: 0,05

Selon le même procédé mais en condensant les halogénures d'alcoyles appropriés ont été obtenus les dérivés suivants illustrant les cas où R= alcoylcarbonyl, alcoyl,

EXEMPLE 2

N-acétonyl-2 oxo-3 p-hydroxyphényl-5 as triazine

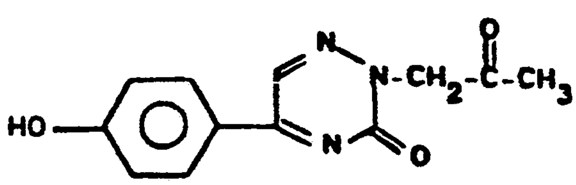

caractéristiques :

– cristaux jaunes

– point de fusion 201°C

– Soluble à chaud, recristallise à froid dans les alcools ; soluble dans les bases diluées aqueuses ; assez soluble dans l'eau.

– Insoluble dans l'éther, le chloroforme, le benzène.

EXEMPLE 3

Acétonyl-2 oxo-3 p (αoxy γ butyrolactone) phényl-5 as triazine

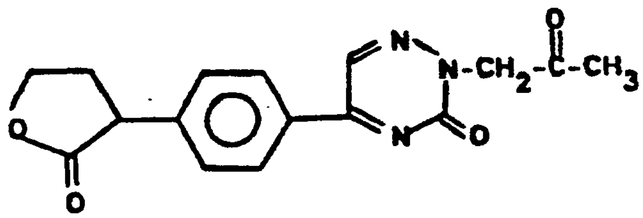

caractéristiques :

- cristaux blancs
- point de fusion 199°C
- Insoluble dans l'eau, le benzène, éther, le chloroforme

EXEMPLE 4

α méthylacétonyl-2 oxo-3 p-méthoxyphényl-5 as triazine

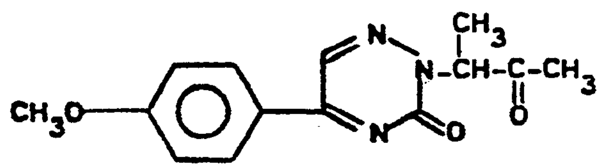

Caractéristiques : Point de fusion : 158°C

- Cristaux beiges
- Soluble dans le chlorure de méthylène, le chloroforme, insoluble dans l'eau

EXEMPLE 5

(méthyl-3′ one-2′ butanyl)-2 oxo-3 p-méthoxyphényl-5 as triazine

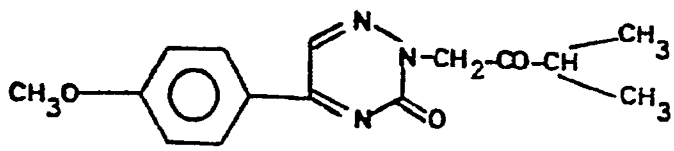

Caractéristiques :

- Cristaux jaunes pâles
- Point de fusion : 168°C ± 2°
- Soluble dans le chloroforme, le chlorure de méthylène insoluble dans l'eau ; peu soluble dans le benzène et l'éther

EXEMPLE 6

acétonyl-2 oxo-3 p-méthylphényl-5 as triazine

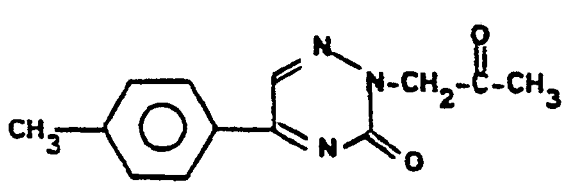

Caractéristiques :

– Cristaux beiges
– Point de fusion : 171°C
– Soluble dans le chloroforme, le chlorure de méthylène ; insoluble dans l'eau, l'éther

EXEMPLE 7

(propyl-3′ one-2′ hexanyl)-2 oxo-3 p-méthoxyphényl-5 as triazine

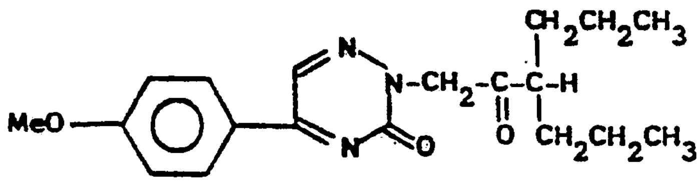

Caractéristiques :
– Cristaux blanc-cassé
– Point de fusion : 181°C
– Soluble dans le chloroforme, le chlorure de méthylène ; insoluble dans l'eau

De la même manière, en traitant avec un halogénure d'alcoyle $CH_3X$ par exemple, le composé de l'exemple 9 obtenu illustre le cas où : **R = alcoyl.**

EXEMPLE 8

méthyl-2 oxo-3 p-acétonoxyphényl-5 as triazine

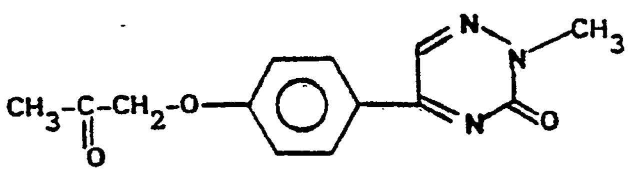

Caractéristiques :
– Cristaux beiges
– Point de fusion : 181°C
– Soluble dans les acides dilués aqueux, et le chlorure de méthylène ; insoluble dans l'eau, le chloroforme

En opérant comme précédemment mais en traitant les intermédiaires triaziniques par des composés de formule :

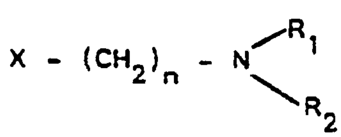

nous avons obtenu les composés suivants :

EXEMPLE 9

β éthyl (métachlorophényl) pipérazine-2 oxo-3 p-méthoxyphényl-5 as triazine

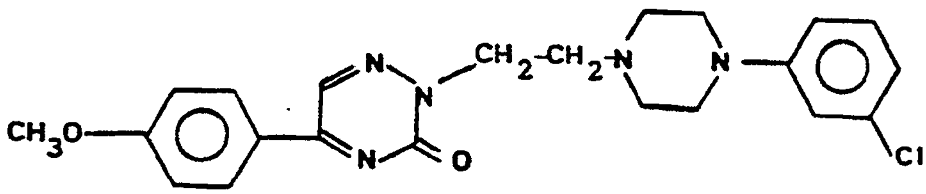

Caractéristiques :

- Cristaux blancs
- Point de fusion : 183°C
- Soluble à chaud dans les acides dilués aqueux
- Peu soluble dans le chloroforme, le chlorure de méthylène

EXEMPLE 10

γ propyl p-pipérazinoacétophénone-2 oxo-3 p-méthylphényl-5 as triazine

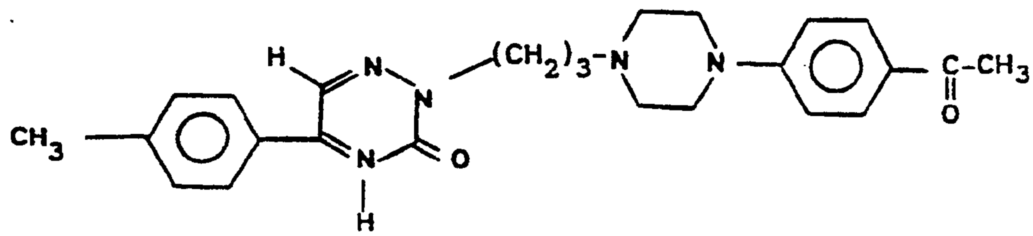

Caractéristiques :

- Cristaux jaunes
- Point de fusion : 141°C
- Soluble à chaud dans les alcools, le chloroforme, le chlorure de méthylène ; insoluble dans l'eau, l'alcool, l'éther

EXEMPLE 11

N- γ propyl (m-chlorophénylpipérazine)-2 oxo-3 dihydro-4,5 p-méthoxyphényl-5 as triazine

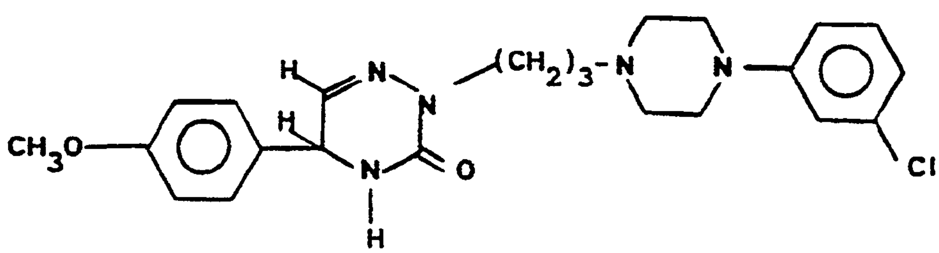

Caractéristiques :

- Cristaux blancs
- Point de fusion : 130°C
- Soluble dans le chloroforme, le chlorure de méthylène, à chaud dans les acides dilués aqueux et les alcools ; insoluble dans l'eau, l'éther

EXEMPLE 12

N- δ butyl (m-chlorophényl pipérazine)-2 oxo-3 p-méthylphényl-5 as triazine

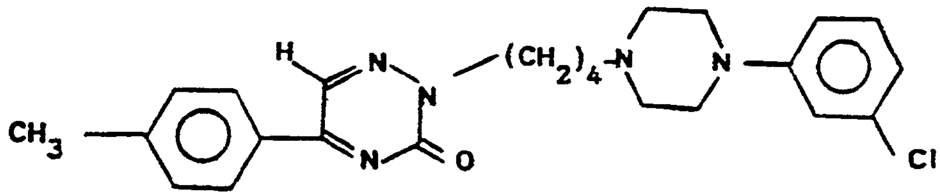

Caractéristiques :

- Cristaux beiges
- Point de fusion : 162°C
- Soluble dans le chloroforme, le chlorure de méthylène ; à chaud dans les alcools et les acides dilués aqueux ; insoluble dans l'eau, l'éther

EXEMPLE 13

chlorhydrate de γ (m-chlorophénylpipérazine propyl-2 oxo-3 p-méthylphényl-5 as triazine

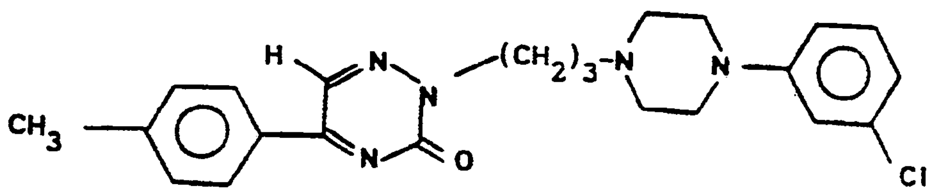

, HCl

Caractéristiques :

- Cristaux blancs
- Point de fusion : ≃ 230°C
- Soluble dans le chloroforme, le chlorure de méthylène ; insoluble dans l'eau, l'éther

EXEMPLE 14

N(ε pentyl m-chlorophényl pipérazine)-2 oxo-3 p-méthoxyphényl-5 as triazine

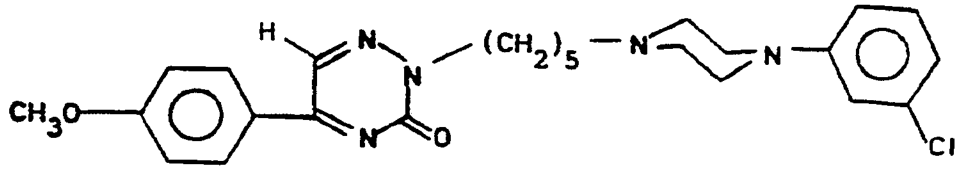

Caractéristiques :

- Cristaux beiges
- Point de fusion : 110°C
- Soluble dans le chloroforme, le chlorure de méthylène ; insoluble dans l'eau, l'éther

EXEMPLE 15

β éthyl (m-trifluorométhylphényl) pipérazine-2 oxo-3 p-méthoxyphényl-5 as triazine

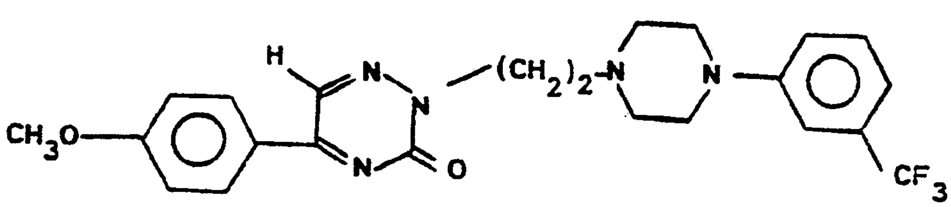

Caractéristiques :

- Cristaux beiges
- Point de fusion : 168°C
- Soluble dans le chloroforme, le chlorure de méthylène ; peu soluble dans le benzène ; insoluble dans l'eau, l'éther

EXEMPLE 16

γ diméthylaminopropyl-2 oxo-3 p-méthoxyphényl-5 as triazine

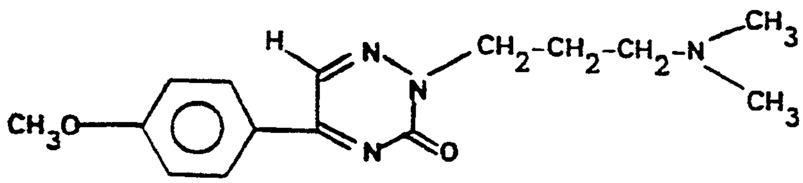

Caractéristiques :

- Cristaux beiges
- Point de fusion 102°C
- Soluble dans le chloroforme, le chlorure de méthylène, le benzène, les alcools, les acides dilués aqueux ; insoluble dans l'éther, l'eau A titre d'exemples non limitatifs, nous citons quelques dérivés qui illustrent divers substituants R fonctionnalisés : acides-alcool en position 2.

EXEMPLE 17

[3-(carboxy-3 propanol-1)]-2 oxo-3 p-méthoxyphényl-5 as triazine

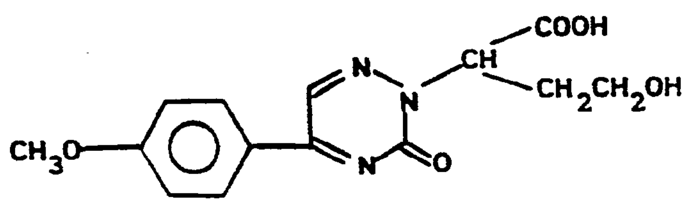

Caractéristiques :

- cristaux jaune-pâle
- Point de fusion : 190°C dec.
- Peu soluble dans le chloroforme, le chlorure de méthylène ; assez soluble dans les alcools ; insoluble dans l'eau

EXEMPLE 18

[3-(carboxy-3 propanol-1)]-2 oxo-3 p-méthylphényl-5 as triazine

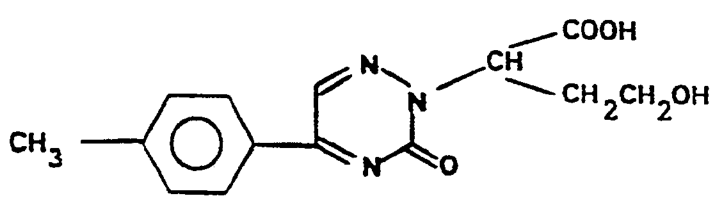

Caractéristiques :

- Cristaux jaune-pâle

- Point de fusion : 180°C dec.
- Peu soluble dans les alcools, le benzène, le chloroforme ; insoluble dans l'eau, l'éther

les lactones correspondantes :

EXEMPLE 19

butyrolacton-2 oxo-3 p-méthylphényl-5 as triazine

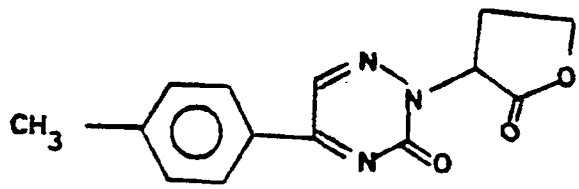

Caractéristiques :
- Cristaux beige-pâle
- Point de fusion : 185°C (EtOH)
- Soluble dans le chloroforme, le chlorure de méthylène ; peu soluble dans l'éther, le benzène ; insoluble dans l'eau,

obtenu à partir du composé de l'exemple 19, en milieu acide

EXEMPLE 20

γ butyrolactone-2 oxo-3 p-méthoxyphényl-5 as triazine

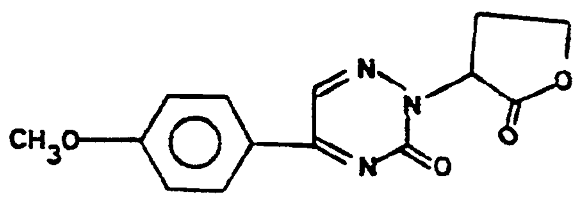

Caractéristiques :
- Cristaux jaune-pâle
- Point de fusion : 194°C (DMF)
- Soluble dans le chloroforme, le chlorure de méthylène ; peu soluble dans le benzène, les alcools ; insoluble dans l'eau

Ce produit a été obtenu à partir de p-méthoxyphényl-5 as triazine par activation de l'azote 2 du cycle triazinique au moyen de l'hydrure de sodium dans le DMF puis condensation avec la α bromo butyrolactone dans le même milieu.

Les exemples précédents ont illustré le cas de Ar, groupe phényl para substitué par un groupe méthyl ou méthoxy ; les exemples suivants complètent les différentes significations objet de la présente invention :

* phényl non substitué (exemple 22)
* orthométhoxy phényl (exemple 23)
* p-éthoxyphényl (exemple 24)
* diméthoxy 2'-4' phényl (exemple 25)

EXEMPLE 21

(méthyl-3 one-2 butanyl)-2 phényl-5 as triazine

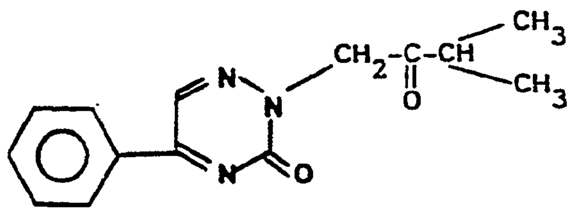

Caractéristiques :

- Cristaux beiges
- Point de fusion : 174°C
- Soluble dans le chloroforme, le chlorure de méthylène ; soluble à chaud dans les alcools ; insoluble dans l'eau

EXEMPLE 22

acétonyl-2 oxo-3 o-méthoxyphényl-5 as triazine

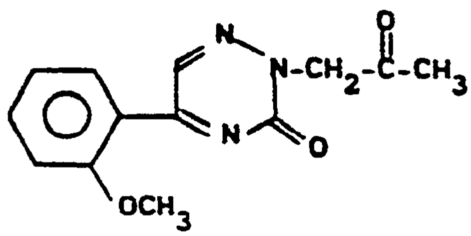

Caractéristiques : Cristaux beiges

- Point de fusion : 125°C
- Soluble dans le chloroforme, le chlorure de méthylène ; insoluble dans l'eau, l'éther

EXEMPLE 23

acétonyl-2 oxo-3 p-éthoxyphényl-5 as triazine

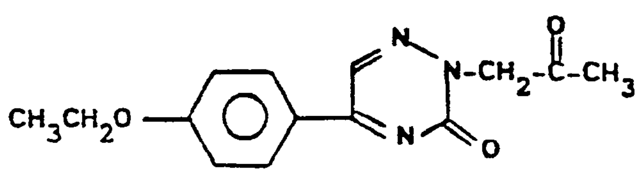

Caractéristiques :

- Cristaux blancs
- Point de fusion : 162°C
- Soluble dans les solvants chlorés ; insoluble dans l'eau, l'éther ; soluble à chaud dans les alcools

EXEMPLE 24

acétonyl-2 oxo-3 (2'-4' diméthoxy)phényl-5 as triazine

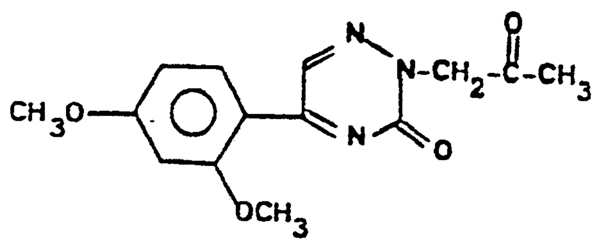

Caractéristiques :
- Cristaux beiges
- Point de fusion : 140° C
- Soluble dans le chloroforme, le chlorure de méthylène ; insoluble dans l'eau, l'éther

EXEMPLE 25

acétonyl-2 oxo-3 α thiényl-5 as triazine

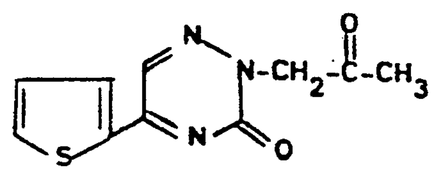

Caractéristiques :
- Cristaux orangés
- Point de fusion : 160°C
- Soluble dans les solvants chlorés insoluble dans l'eau, l'éther

Enfin les derniers exemples illustrent des substituants R diversement fonctionnalisés :

EXEMPLE 26

diéthoxysuccinyl-2 oxo-3 p-méthoxyphényl-5 as triazine

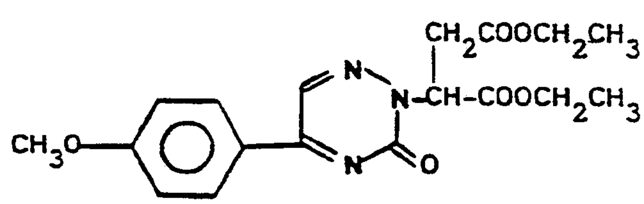

Caractéristiques :
- Cristaux blancs
- Point de fusion : 78°C
- Soluble dans le benzène, les alcools, le chloroforme ; insoluble dans l'eau

EXEMPLE 27

N [ ε pentyl-(4-hydroxypipéridine)]-2 oxo-3 p-méthoxyphényl-5 as triazine

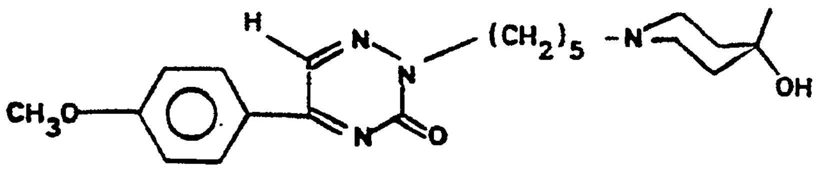

Caractéristiques :

- Cristaux beiges
- Point de fusion : 114°C
- Soluble dans les alcools, le chloroforme ; insoluble dans l'eau

EXEMPLE 28

N [α (1,2 diphényléthane ol-1 )]-2 oxo-3 dihydro 4,5 p-méthylphényl-5 as triazine

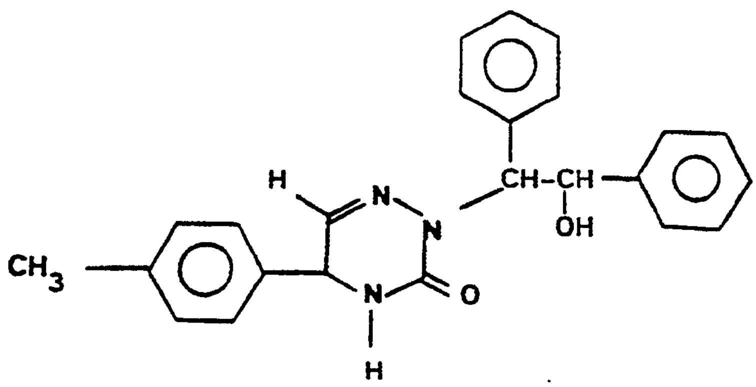

Caractéristiques :

- Cristaux blancs
- Point de fusion : 214°C
- Soluble dans les alcools à chaud ; insoluble dans l'eau, l'éther, le chloroforme

EXEMPLE 29

N-diphénylméthyl-2 oxo-3 p-méthoxyphényl-5 as triazine

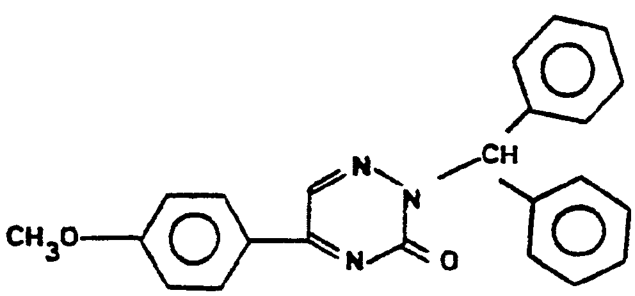

Caractéristiques :

- Cristaux beiges
- Point de fusion : 193°C
- Soluble dans les alcools à chaud, le chlorure de méthylène insoluble dans l'eau, le chloroforme

EXEMPLE 30

éthanol-2 oxo-3 p-méthoxyphényl-5 as triazine

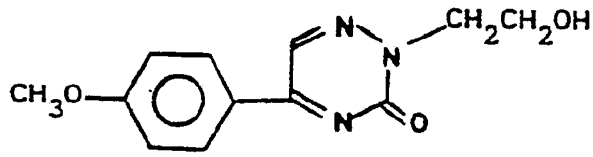

Caractéristiques :
- Cristaux beiges
- Point de fusion : 134°C
- Soluble à chaud dans l'eau, peu soluble dans le chloroforme ; insoluble dans le benzène, l'éther ; assez soluble dans le chlorure de méthylène

EXEMPLE 31

N-acétamido-2 oxo-3 p-méthoxyphényl-5 as triazine

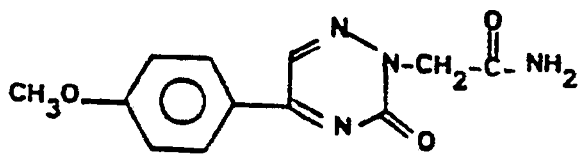

Caractéristiques :
- Cristaux jaunes
- Point de fusion : 268°C
- Insoluble dans l'eau, le chloroforme, l'éther, les alcools

EXEMPLE 32

N ( γ p-fluorobutyrophénone)-2 oxo-3 p-méthoxyphényl-5 as triazine

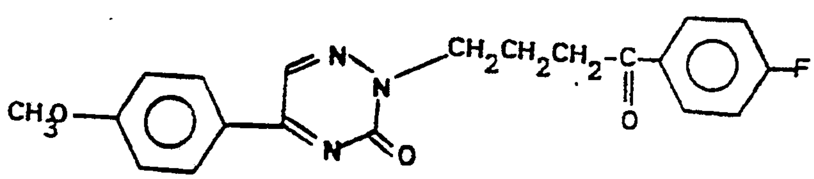

Caractéristiques :
- Cristaux blancs
- Point de fusion : 156°C
- Soluble dans le chloroforme, le chlorure de méthylène ; insoluble dans l'eau

EXEMPLE 33

N-désyl-2 oxo-3 p-méthoxyphényl-5 as triazine

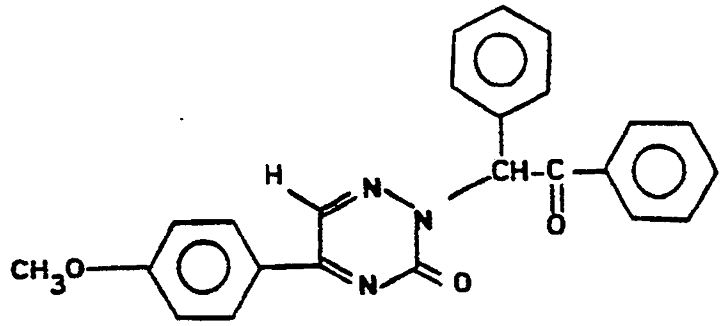

Caractéristiques :
- Cristaux beiges
- Point de fusion : 190°C
- Soluble à chaud dans les alcools ; assez soluble dans le chloroforme ; peu soluble dans l'éther ; insoluble dans l'eau

EXEMPLE 34

N-éthylbutyrate-2 oxo-3 p-méthylphényl-5 as triazine

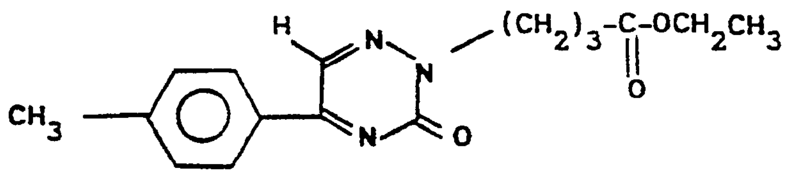

Caractéristiques :
- Cristaux blancs
- Point de fusion : 52°C
- Soluble dans le benzène, les alcools, le chloroforme ; insoluble dans l'eau

EXEMPLE 35

N-désyl-2 oxo-3 p-méthylphényl-5 as triazine

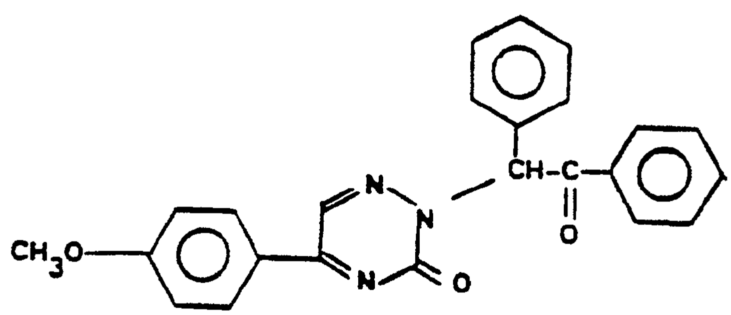

Caractéristiques :
- Cristaux jaune-pâle
- Point de fusion : 215°C
- Soluble dans les alcools à chaud, le chloroforme, le chlorure de méthylène ; insoluble dans l'eau.

EXEMPLE 36

Phénacyl-2 oxo-3 p-méthoxyphényl-5 as triazine

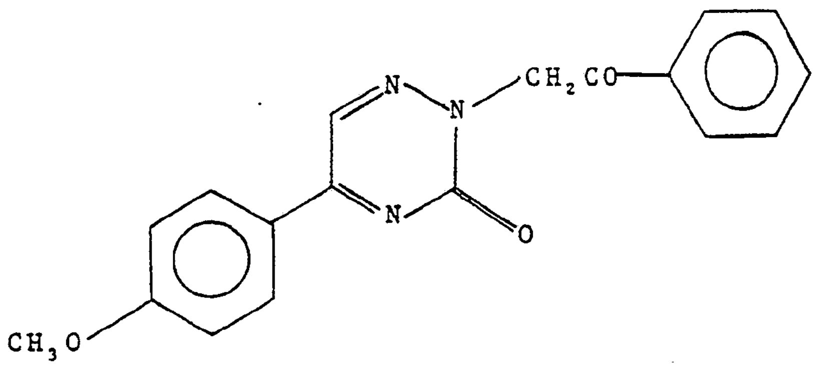

Caractéristiques :
- Point de fusion : 210°C
- Soluble dans l'acide acétique ; insoluble dans l'eau

EXEMPLE 37

p-méthylphénacyl-2 oxo-3 p-méthoxyphényl-5 as triazine

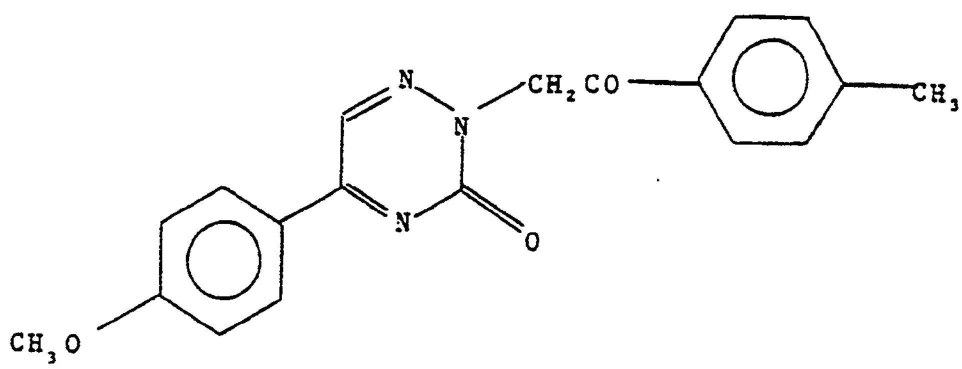

Caractéristiques :
- Point de fusion : 196°C
- Soluble dans l'acide acétique ; insoluble dans l'eau et dans les alcools

EXEMPLE 38

(dichloro-2,4 phénacyl)-2 oxo-3 p-méthoxyphényl-5 as triazine

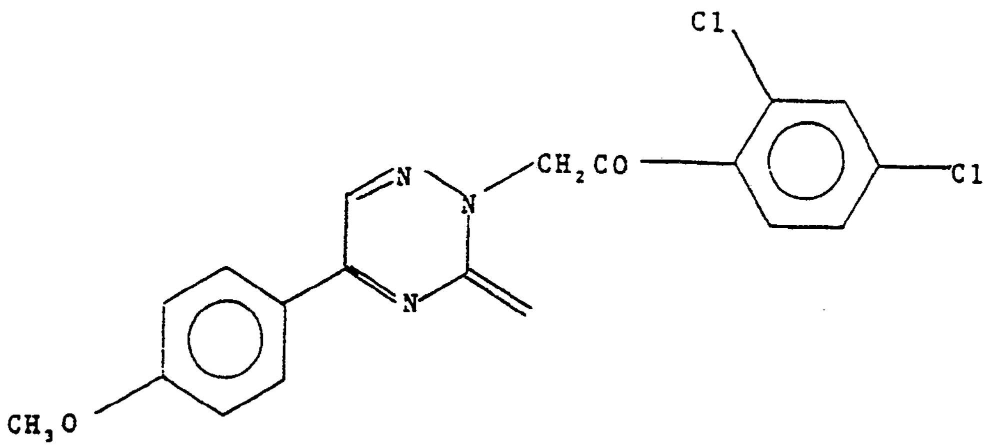

Caractéristiques :
– Point de fusion : 192°C
– Soluble dans l'acide acétique ; insoluble dans l'eau et les alcools

EXEMPLE 39

p-méthoxyphénacyl-2 oxo-3 p-méthoxyphényl-5 as triazine

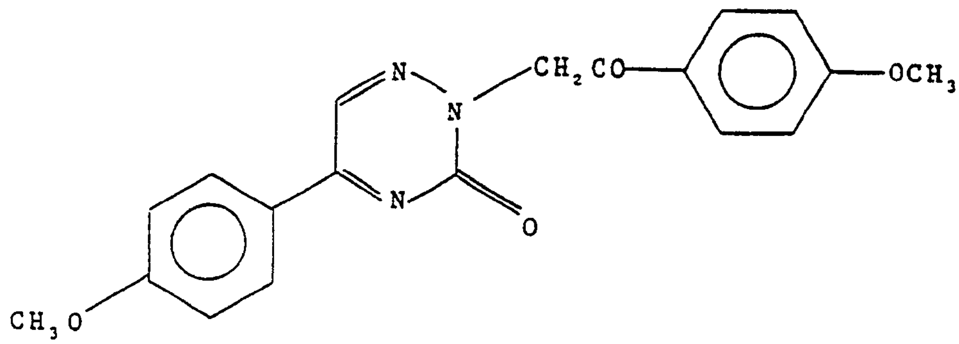

Caractéristiques :
– Point de fusion : 220°C
– Insoluble dans l'eau, l'acide acétique et les alcools

EXEMPLE 40

(oxo-2′ butanyl)-2 oxo-3 p-méthoxyphényl-5 as triazine

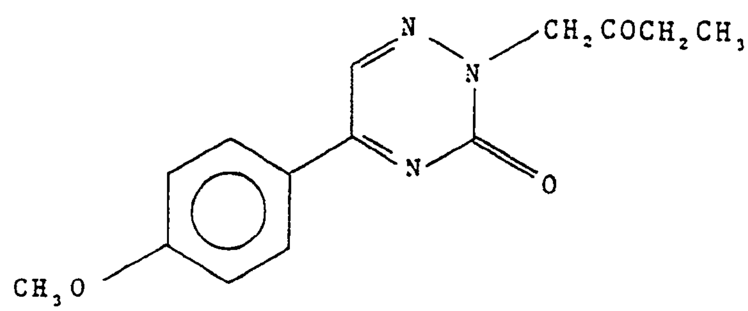

Caractéristiques :
– Point de fusion : 152°C
– Soluble dans l'acide acétique ; insoluble dans l'eau et dans les alcools

EXPERIMENTATIONS PHARMACOLOGIQUES

a) Toxicologie

L'étude de toxicité a été effectuée chez la souris conventionnelle pesant environ 20 g.

Les substances ont été administrées par voie orale en dose unique respectivement 300 et 1000 mg/kg. A ces doses, nous n'avons pas pu déterminer les $DL_{50}$ qui sont supérieures : 1000 mg/kg. A titre d'exemple pour le composé de l'exemple 1, la $DL_{50}$ est supérieure à 3000 mg/kg.

b) Action sur le système nerveux central

Les essais retenus pour le triage pharmacologique sont :

la potentialisation de 5 HTP (réf.:Christensen A.V. et al., Eur. J. of Pharm. (1977) 41,153-162)

la potentialisation de 1-DOPA (réf. Turner and Hebborn, Screening methods in pharmacology, vol. II ( 1971 ) Academic Press (New York and London)).

Les résultats pour quelques dérivés parmi les plus actifs figurent dans le tableau ci-dessous :

| Composé de l'exemple n° | Pot. 5 HTP $ED_{50}$ en mg/kg | Pot. 1-DOPA $ED_{50}$ salivation |
|---|---|---|
| 1 | 25 | 50 |
| 4 | 35 | 60 |
| 8 | 40 | 100 |
| 20 | 27 | 70 |
| 22 | 50 | 85 |
| 31 | 20 | 25 |

Ces composés ont également été étudiés pour rechercher une activité anxiolytique en utilisant un ensemble d'essais

(Réf.: LAB-X : PELLOW et al., ....
SKINNER : GELLER and SEIFTER, Psychopharmacologia (1960) 1, 482-492
VOGEL : VOGEL J.R., BEER B., CLODY D.E., Psychopharmacologia (1971), 21 1-7),

les composés sont testés à 30 mg/kg per os, des résultats significatifs ont été trouvés pour les composés des exemples 1,4,9, 21 et 32.

En conclusion, pour ce qui concerne les applications thérapeutiques des compositions pharmaceutiques selon la présente invention, compte tenu des effets observés en pharmacologie et de la très faible toxicité des composés les plus actifs et plus particulièrement les composés des exemples 1,4,21 et 32 peuvent être utilisés pour traiter les troubles liés à l'anxiété et/ou aux états dépressifs.

Ces composés sont en outre dépourvus d'effets sédatifs, ce qui les particularise par rapport aux anxiolytiques diazépiniques.

## Revendications

1. Monoaryl-5 as triazinones-3 substituées en position 2 répondant à la formule générale (I)

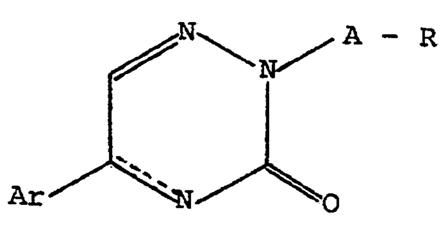

(I)

dans laquelle :

la liaison représentée par des tirets indique la présence d'une double liaison facultative ;

A représente une liaison directe N-C, un alkylène droit ou ramifié en $C_1$ à $C_5$, éventuellement substitué une ou plusieurs fois par -COOR' ou par Ar ;

R représente H, -OH, 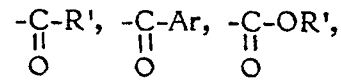

$-NR'_2$, 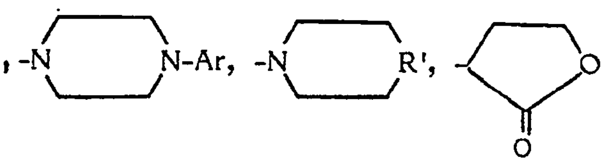

R' représente -H, alkyl droit ou ramifié en $C_1$ à $C_7$, $-NH_2$ ;

Ar représente un noyau aromatique à 5 ou 6 chaînons, contenant éventuellement un hétéroatome tel que O, N, S et obligatoirement substitué une ou plusieurs fois par un radical choisi parmi -OH, alkyl en $C_1$ à $C_4$, alcoxy en $C_1$ à $C_4$, halogène, $-CF_3$, acétonyloxy,

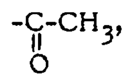

et γ butyrolactone ;
ainsi que leurs sels pharmaceutiquement acceptables.

2. Monoaryl-5 triazinones-3 substituées en position 2, selon la revendication 1, de formule générale I dans laquelle:

Ar représente

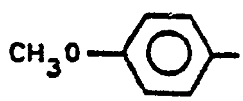

3. Les composés chimiques selon la revendication 1, et plus particulièrement :

. acétonyl-2 oxo-3 p-méthoxyphényl-5 as triazine
. N-acétonyl-2 oxo-3 p-hydroxyphényl-5 as triazine
. Acétonyl-2 oxo-3 p(α oxy γ butyrolactone) phényl-5 as triazine
. α méthylacétonyl-2 oxo-3 p-méthoxyphényl-5 as triazine
. (méthyl-3′ one-2′ butanyl)-2 oxo-3 p-méthoxyphényl-5 as triazine
. acétonyl-2 oxo-3 p-méthylphényl-5 as triazine
. acétonyl-2 oxo-3 phényl-5 as triazine
. (propyl-3′ one-2′ hexanyl)-2 oxo-3 p-méthoxyphényl-5 as triazine
. méthyl-2 oxo-3 p-acétonoxyphényl-5 as triazine
. β éthyl (métachlorophényl) pipérazine-2 oxo-3 p-méthoxyphényl-5 as triazine
. γ propyl p-pipérazinoacétophénone-2 oxo-3 p-méthylphényl-5 as triazine
. N- γ propyl (m-chlorophénylpipérazine)-2 oxo-3 dihydro-4,5 p-méthoxyphényl-5 as triazine
. N- δ butyl (m-chlorophényl pipérazine)-2 oxo-3 p-méthylphényl-5 as triazine
. Chlorhydrate de γ (m-chlorophénylpipérazine propyl-2 oxo-3 p-méthylphényl-5 as triazine
. N(ε pentyl m-chlorophényl pipérazine)-2 oxo-3 p-méthoxyphényl-5 as triazine
. β éthyl (m-trifluorométhylphényl) pipérazine-2 oxo-3 p-méthoxyphényl-5 as triazine
. γ diméthylaminopropyl-2 oxo-3 p-méthoxyphényl-5 as triazine
. [3-(carboxy-3 propanol-1)]-2 oxo-3 p-méthoxyphényl-5 as triazine
. [3-(carboxy-3 propanol-1)]-2 oxo-3 p-méthylphényl-5 as triazine
. butyrolacton-2 oxo-3 p-méthylphényl-5 as triazine
. γ butyrolactone-2 oxo-3 p-méthoxyphényl-5 as triazine
. (méthyl-3 one-2 butanyl)-2 phényl-5 as triazine
. acétonyl-2 oxo-3 o-méthoxyphényl-5 as triazine
. acétonyl-2 oxo-3 p-éthoxyphényl-5 as triazine
. acétonyl-2 oxo-3 (2′-4′ diméthoxy)phényl-5 as triazine
. acétonyl-2 oxo-3 α thiényl-5 as triazine
. diéthoxysuccinyl-2 oxo-3 p-méthoxyphényl-5 as triazine

. N [ ε pentyl-(4-hydroxypipéridine)]-2 oxo-3 p-méthoxyphényl-5 as triazine
. **N [α (1,2 diphényléthane ol-1)]-2 oxo-3 dihydro 4,5 p-méthylphényl-5 as triazine**
. N-diphénylméthyl-2 oxo-3 p-méthoxyphényl-5 as triazine
. éthanol-2 oxo-3 p-méthoxyphényl-5 as triazine
. N-acétamido-2 oxo-3 p-méthoxyphényl-5 as triazine
. N (γ p-fluorobutyrophénone)-2 oxo-3 p-méthoxyphényl-5 as triazine
. N-désyl-2 oxo-3 p-méthoxyphényl-5 as triazine
. N-éthylbutyrate-2 oxo-3 p-méthylphényl-5 as triazine
. N-désyl-2 oxo-3 p-méthylphényl-5 as triazine.
. Phénacyl-2 oxo-3 p-méthoxyphényl-5 as triazine
. p-méthylphénacyl-2 oxo-3 p-méthoxyphényl-5 as triazine
. (dichloro-2,4 phénacyl)-2 oxo-3 p-méthoxyphényl-5 as triazine
. p-méthoxyphénacyl-2 oxo-3 p-méthoxyphényl-5 as triazine
. (oxo-2′ butanyl)-2 oxo-3 p-méthoxyphényl-5 as triazine.

4. Procédé de préparation des composés de formule générale (I) selon l'une des revendications 1 à 3, caractérisé en ce que l'on fait réagir un composé de formule générale (II)

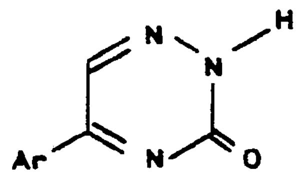

(II)

en présente d'un solvant approprié et d'un agent sodant, tel qu'un hydrure ou un amidure de métal alcalin, avec un réactif de formule générale (III)

Hal - A - R (III)

formules dans lesquelles Ar, A et R ont les significations données à la revendication 1, et Hal représente un atome d'halogène, tel que le chlore ou le brome.

5. Procédé selon la revendication 4, caractérisé en ce que l'agent sodant utilisé est un hydrure ou un amidure de métal alcalin, en particulier l'hydrure ou l'amidure de sodium.

6. Procédé selon l'une des revendications 4 ou 5, caractérisé en ce que le solvant utilisé est le DMF.

7. Compositions pharmaceutiques, utiles en particulier pour le traitement des maladies du système nerveux central, comportant, à titre de principe actif au moins l'un des composés selon l'une des revendications 1 à 3, et à titre de support ou de vecteur un excipient pharmaceutiquement acceptable.

8. Utilisation d'au moins l'un des composés selon l'une des revendications 1 à 3, pour la préparation de compositions pharmaceutiques utiles pour le traitement des maladies du système nerveux central.

## Patentansprüche

1. In 2-Stellung substituierte 5-Monoaryl-as-triazin-3-one der allgemeinen Formel (I)

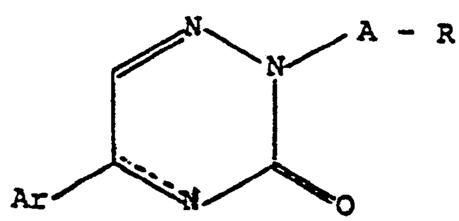

(I)

worin darstellen

die gestrichelt gezeichnete Linie das Vorliegen einer fakultativen Doppelbindung anzeigt

A eine direkte N-C-Bindung, ein gerades oder verzweigtes $C_1$-$C_5$-Alkylen, das gegebenenfalls einfach oder mehrfach durch -COOR′ oder durch Ar substituiert ist;

R 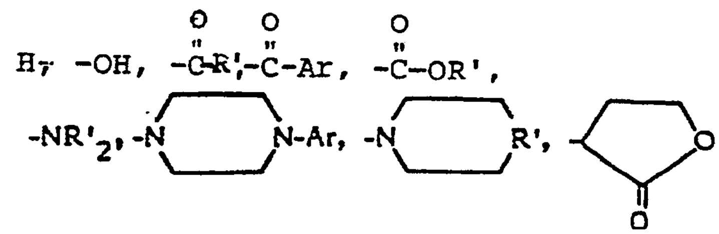

worin R' H, gerades oder verzweigtes $C_1$-$C_7$-Alkyl oder $-NH_2$ darstellt;

Ar einen 5- oder 6-gliedrigen aromatischen Ring, der gegebenenfalls ein Heteroatom wie O, N, S enthält und zwingend ein- oder mehrfach substituiert ist durch einen Rest, ausgewählt aus -OH, $C_1$-$C_4$-Alkyl, $C_1$-$C_4$-Alkoxy, Halogen, $-CF_3$, Acetonyloxy,

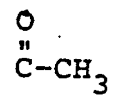

und γ-Butyrolacton;
sowie ihre pharmazeutisch akzeptablen Salze.

2. In 2-Stellung substituierte 5-Monoaryl-as-triazin-3-one der allgemeinen Formel (I), worin Ar darstellt

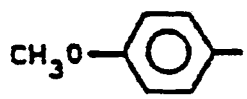

3. Die chemischen Verbindungen nach Anspruch 1 und insbesondere:
. 2-Acetonyl-3-oxo-5-p-methoxyphenyl-as-triazin
. 2-N-Acetonyl-3-oxo-5-p-hydroxyphenyl-as-triazin
. 2-Acetonyl-3-oxo-5-p(α-oxy-γ-butyrolacton)phenyl-as-triazin
. 2-α-Methylacetonyl-3-oxo-5-p-methoxyphenyl-as-triazin
. 2-(3'-Methyl-2'-on-butanyl)-3-oxo-5-p-methoxyphenyl-as-triazin
. 2-Acetonyl-3-oxo-5-p-methylphenyl-as-triazin
. 2-Acetonyl-3-oxo-5-phenyl-as-triazin
. 2-(3'-Propyl-2'-on-hexanyl)-3-oxo-5-p-methoxyphenyl-as-triazin
. 2-Methyl-3-oxo-5-p-acetonoxyphenyl-as-triazin
. 2-β-Ethyl-(m-chlorophenyl)piperazin-3-oxo-5-p-methoxyphenyl-as-triazin
. 2-γ-Propyl-p-piperazinoacetophenon-3-oxo-5-p-methylphenyl-as-triazin
. 2-N- γ-Propyl-(m-chlorophenylpiperazin)-3-oxo-4,5-dihydro-5-p-methoxyphenyl-as-triazin
. 2-N-δ-Butyl-(m-chlorophenyl-piperazin)-3-oxo-5-p-methylphenyl-as-triazin
. Hydrochlorid von 2-γ-(m-Chlorophenylpiperazin)-propyl-3-oxo-5-p-methylphenyl-as-triazin
. 2-N-(ε-Pentyl-m-chlorophenyl-piperazin)-3-oxo-5-p-methoxyphenyl-as-triazin
. 2-β-Ethyl-(m-trifluoromethylphenyl)-piperazin-3-oxo-5-p-methoxyphenyl-as-triazin
. 2-γ-Dimethylaminopropyl-3-oxo-5-p-methoxyphenyl-as-triazin
. 2-[3-(3-Carboxy-1-propanol)]-3-oxo-5-p-methoxyphenyl-as-triazin
. 2-[3-(3-Carboxy-1-propanol)]-3-oxo-5-p-methylphenyl-as-triazin
. 2-Butyrolacton-3-oxo-5-p-methylphenyl-as-triazin
. 2-γ-Butyrolacton-3-oxo-5-p-methoxyphenyl-as-triazin
. 2-(3-Methyl-2-on-butanyl)-5-phenyl-as-triazin
. 2-Acetonyl-3-oxo-5-o-methoxyphenyl-as-triazin
. 2-Acetonyl-3-oxo-5-p-ethoxyphenyl-as-triazin
. 2-Acetonyl-3-oxo- 5-(2',4'-dimethoxy)phenyl-as-triazin
. 2-Acetonyl-3-oxo-5-α-thienyl-as-triazin
. 2-Diethoxysuccinyl-3-oxo-5-p-methoxyphenyl-as-triazin
. 2-N-[ε-Pentyl-4-hydroxypiperidin]-3-oxo-5-p-methoxyphenyl-as-triazin
. 2-N-[α-(1,2-Diphenylethan-1-ol)]-3-oxo-4,5-dihydro-5-p-methylphenyl-as-triazin
. 2-N-Diphenylmethyl-3-oxo-5-p-methoxyphenyl-as-triazin

. 2-Ethanol-3-oxo-5-p-methoxyphenyl-as-triazin
. 2-N-Acetamido-3-oxo-5-p-methoxyphenyl-as-triazin
. 2-N-(γ-p-Fluorobutyrophenon)-3-oxo-5-p-methoxyphenyl-as-triazin
. 2-N-Desyl-3-oxo-5-p-methoxyphenyl-as-triazin
. 2-N-Ethylbutyrat-3-oxo-5-p-methylphenyl-as-triazin
. 2-N-Desyl-3-oxo-5-p-methylphenyl-as-triazin
. 2-Phenacyl-3-oxo-5-p-methoxyphenyl-as-triazin
. 2-p-Methylphenacyl-3-oxo-5-p-methoxyphenyl-as-triazin
. 2-(2,4-Dichloro-phenacyl)-3-oxo-5-p-methoxyphenyl-as-triazin
. 2-p-Methoxyphenacyl-3-oxo-5-p-methoxyphenyl-as-triazin
. 2-(2'-Oxo-butanyl)-3-oxo-5-p-methoxyphenyl-as-triazin.

4. Verfahren zur Herstellung der Verbindungen der allgemeinen Formel (I) nach einem der Ansprüche 1 bis 3, dadurch gekennzeichnet, daß man eine Verbindung der allgemeinen Formel (II)

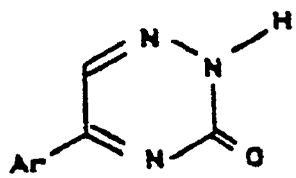

(II)

in Gegenwart eines geeigneten Lösungsmittels und eines Salzbildenden Agens, wie z.B. eines Alkalimetallhydrids oder -amids, mit einem Reagens der allgemeinen Formel (III) reagieren läßt

Hal - A - R (III)

wobei in den obengenannten Formeln Ar, A und R die in Anspruch 1 angegebenen Bedeutungen haben und Hal ein Halogenatom, wie z.B. Chlor oder Brom, bedeutet.

5. Verfahren nach Anspruch 4, dadurch gekennzeichnet, daß als Salz-bildendes Agens ein Alkalimetallhydrid oder -amid, insbesondere Natriumhydrid oder -amid, verwendet wird.

6. Verfahren nach einem der Ansprüche 4 oder 5, dadurch gekennzeichnet, daß als Lösungsmittel DMF verwendet wird.

7. Pharmazeutische Zusammensetzungen, die insbesondere geeignet sind für die Behandlung von Erkrankungen des Zentralnervensystems, die als aktives Prinzip (Wirkstoff) mindestens eine der Verbindungen nach einem der Ansprüche 1 bis 3 und als Träger oder Vektor einen pharmazeutisch akzeptablen Exzipienten enthalten.

8. Verwendung mindestens einer der Verbindungen nach einem der Ansprüche 1 bis 3 zur Herstellung von pharmazeutischen Zusammensetzungen, die geeignet sind für die Behandlung von Erkrankungen des Zentralnervensystems.

## Claims

1. 5-monoaryl-3-as-triazinones substituted at the 2-position, corresponding to the general formula (I) :

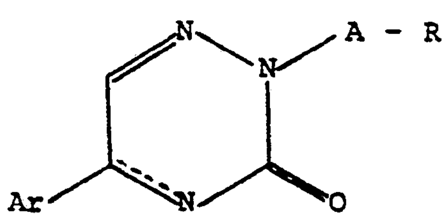

(I)

in which :

the bond represented by dashes indicates the presence of an optional double bond ;

A denotes a direct N-C bond, or a linear or branched $C_1$ to $C_5$ alkylene optionally substituted one or more times with -COOR' or with Ar ;

R denote -H, -OH, 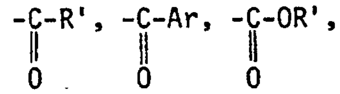

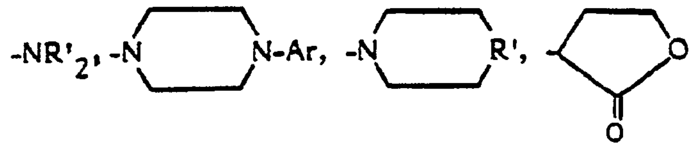

R′ denotes -H, linear or branched $C_1$ to $C_7$ alkyl, $-NH_2$ ;

Ar denotes a 5- or 6-membered aromatic ring optionally containing a hetero atom such as O, N, S, and compulsorily substituted one or more times with a radical chosen from -OH, $C_1$ to $C_4$ alkyl, $C_1$ to $C_4$ alkoxy, halogen, $-CF_3$, acetonyloxy,

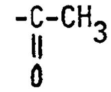

and γ-butyrolactone ;

as well as their pharamceutically acceptable salts.

2. 5-Monoaryl-3-as-triazinones substituted at the 2-position according to Claim 1, of general formula I, in which :

Ar denotes

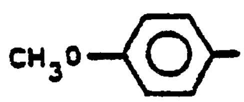

3. The chemical compounds according to Claim 1, and more especially :

2-acetonyl-3-oxo-5-(p-methoxyphenyl)-as-triazine

N2—acetonyl-3-oxo-5-(p-hydroxyphenyl)-as-triazine

. 2-acetonyl-3-oxo-5-[p-oxotetrahydrofur-3-yl)phenyl]-as-triazine

. 2-(α-methylacetonyl)-3-oxo-5-(p-methoxyphenyl)-as-triazine

. 2-(3-methyl-2-oxobutyl)-3-oxo-5-(p-methoxyphenyl)-as-triazine

. 2-acetonyl-3-oxo-5-(p-methylphenyl)-as-triazine

. 2-(3-propyl-2-oxohexyl)-3-oxo-5-(p-methoxyphenyl)-as-triazine

. 2-methyl-3-oxo-5-(p-acetonyloxyphenyl)-as-triazine

. 2-{β-[4-(meta-chlorophenyl)-1-piperazinyl]ethyl}-3-oxo-5-(p-methoxyphenyl)-as-triazine

. 2-{γ-[4-)p-acetylphenyl)-1-piperazinyl]propyl}-3-oxo-5-(p-methylphenyl)-as-triazine

. N2-{γ-[4-(m-chlorophenyl)-1-piperazinyl]propyl}-3-oxo-4,5-dihydro-5-(p-methoxyphenyl)-as-triazine

. N2-{δ-[4-(m-chlorophenyl)-1-piperazinyl]butyl}-3-oxo-5-(p-methylphenyl)-as-triazine

. 2-{γ-[4-(m-chlorophenyl)-1-piperazyl]propyl}-3-oxo-5-(p-methylphenyl)-as-triazine hydrochloride

. N2-{ε-[4-(m-chlorophenyl)-1-piperazinyl[pentyl}-3-oxo-5-(p-methoxyphenyl)-as-triazine

. 2-{β-[4-(m-trifluoromethylphenyl)-piperazinyl]ethyl}-3-oxo-5-(p-methoxyphenyl)-as-triazine

. 2-[γ-(dimethylamino)propyl]-3-oxo-5-(p-methoxyphenyl)-as-triazine

. 2-(1-carboxy-3-hydroxypropyl)-3-oxo-5-(p-methoxyphenyl)-as-triazine

. 2-(1-carboxy-3-hydroxypropyl)-3-oxo-5-(p-methylphenyl)-as-triazine

. 2-(2-oxotetrahydrofur-3-yl)-3-oxo-5-(p-methylphenyl)-as-triazine

. 2-(2-oxotetrahydrofur-3-yl)-3-oxo-5-(p-methoxyphenyl)-as-triazine

. 2-(3-methyl-2-oxobutyl)-3-oxo-5-phenyl-as-triazine

. 2-acetonyl-3-oxo-5-(o-methoxyphenyl-as-triazine

. 2-acetonyl-3-oxo-5-(p-ethoxyphenyl)-as-triazine

. 2-acetonyl-3-oxo-5-(2,4-dimethoxyphenyl)-as-triazine

. 2-acetonyl-3-oxo-5-(2-thienyl)-as-triazine

. 2-[1,2-bis(ethoxycarbonyl)ethyl]-3-oxo-5-(p-methoxyphenyl)-as-triazine

. $N^2$-[ε-(4-hydroxypiperidino)pentyl]-3-oxo-5-(p-methoxyphenyl)-as-triazine
. $N^2$-(1,2-diphenyl-2-hydroxyethyl)-3-oxo-4,5-dihydro-5-(p-methylphenyl)-as-triazine
. $N^2$-diphenylmethyl-3-oxo-5-(p-methoxyphenyl)-as-triazine
. 2-(2-hydroxyethyl)-3-oxo-5-(p-methoxyphenyl)-as-triazine
. $N^2$-carbamoylmethyl-3-oxo-5-(p-methoxyphenyl)-as-triazine
. $N^2$-[γ-(p-fluorobenzoyl-propyl]-3-oxo-5-(p-methoxyphenyl)-as-triazine
. $N^2$-desyl-3-oxo-5-(p-methoxyphenyl)-as-triazine
. $N^2$-[γ-(ethoxycarbonyl)propyl]-3-oxo-5-(p-methylphenyl)-as-triazine
. $N^2$-desyl-3-oxo-5-(p-methylphenyl-as-triazine
. 2-phenacyl-3-oxo-5-(p-methoxyphenyl)-as-triazine
. 2-(p-methylphenacyl)-3-oxo-5-(p-methoxyphenyl)-as-triazine
. 2-(2,4-dichlorophenacyl)-3-oxo-5-(p-methoxyphenyl)-as-triazine
. 2-(p-methoxyphenacyl)-3-oxo-5-(p-methoxyphenyl)-as-triazine
. 2-(2-oxobutyl)-3-oxo-5-(p-methoxyphenyl)-as-triazine

4. Process for preparing the compounds of general formula (I) according to one of Claims 1 to 3, characterized in that a compound of general formula (II)

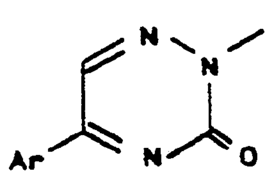

(II)

is reacted, in the presence of a suitable solvent and an alkali-metalating agent such as a hydride or an amide of an alkali metal, with a reagent of general formula (III)

Hal - A- R (III)

in which formulae Ar, A and R have the meanings given in Claim 1, and Hal denotes a halogen atom such as chlorine or bromine.

5. Process according to Claim 4, characterized in that the alkali-metalating agent used is an alkali metal hydride or amide, especially sodium hydride or amide.

6. Process according to one of Claims 4 and 5, characterized in that the solvent used is DMF.

7. Pharmaceutical compositions, especially for the treatment of diseases of the central nervous system, containing at least one of the compounds according to any one of Claims 1 to 3 by way of active principle, and a pharmaceutically acceptable excipient by way of carrier or vector.

8. Use of at least one of the compounds according to any one of Claims 1 to 3, for the preparation of pharmaceutical compositions which are useful for the treatment of diseases of the central nervous system.